# EUROPEAN PATENT APPLICATION

(11) **EP 1 031 353 A1**
(43) Date of publication of application: **30.08.2000**
(21) Application number: 00301479.2
(22) Date of filing: 24.02.2000
(51) Int. Cl.: A61K 47/36, A61K 47/32, A61K 31/4174, A61K 31/27, A61K 31/573, A61P 31/10, A61P 29/00, A61P 31/04

(54) **Antimycotic compositions**

(30) Priority: 24.02.1999 US 257078
(71) Applicant: Stiefel Research Institute, Inc., Coral Gables, FL 33134 (US)
(72) Inventor: Sakai, K. Kirk, Margate, FL 33063 (US); Martinolich, Michael J., Miami, FL33186 (US)
(74) Representative: Howard, Paul Nicholas

(57) **Abstract**

Topical antimycotic compositions contain at least one topically effective antimycotic agent in an amount at least sufficient for produce a topical antimycotic effect, a topically acceptable polyol/acrylic copolymer, and a topically acceptable particulate vegetable starch, in an aqueous alcoholic base. A topical steroidal anti-inflammatory agent and/or a topical antimicrobial agent also may be present. A typical embodiment combines miconazole nitrate in an acrylate/C10-30 alkyl acrylate copolymer and tapioca starch in ethanol and water.

## Description

### Background of the Invention

The present invention relates to topical pharmaceutical compositions that are particularly suitable for treating fungal diseases.

A fungus is a microscopic plant cell that can grow on the skin and, under certain conditions, produce an infection, or mycosis. A fungal infection is commonly associated with signs of erythema and scaling and with symptoms of itching or painful burning. A variety of methods have been used for the treatment of fungal infections including the use of potassium iodide, Whitfield's ointment, undecylenic acid, antibiotics (e.g. nystatin and amphotericin B), griseofulvin, and the imidazole antifungal agents such as miconazole. Clinical treatment requires at least two to four weeks for complete relief of symptoms.

### Detailed Description

The present composition comprises at least one topically effective antimycotic agent in an amount at least sufficient to produce a topical antimycotic effect, in a formulation matrix comprising a topically acceptable polyol/acrylic copolymer and a topically acceptable particulate vegetable starch, in an aqueous alcoholic base. The composition produces a topical antimycotic effect and leaves the skin with a soothing "powdery" after-feel.

The topically effective antimycotic agents include imidazoles (and triazoles) such as miconazole, econazole, clotrimazole, ethonam, terconazole, azaconazole, sulconazole, and ketoconazole, allylamines such as naftifine, pyridine derivatives such as ciclopirox and zinc pyrithione, undecylenic acid derivatives, tolnaftate, haloprogin, clioquinol, acrisorcin, iodochlorhydroxyquin, nystatin.

When capable of forming a salt, the topically effective antimycotic agents can be administered as such. Physiologically acceptable non-toxic salts include those derived from organic and inorganic acids such as, without limitation, hydrochloric acid, hydrobromic acid, nitric acid, phosphoric acid, sulfuric acid, methanesulphonic acid, acetic acid, tartaric acid, lactic acid, succinic acid, citric acid, malic acid, maleic acid, sorbic acid, aconitic acid, salicylic acid, phthalic acid, embonic acid, enanthic acid, and the like. Also included are the physiologically acceptable salts of those topically effective antimycotic agents which are acidic in nature with metals, alkali metals, alkaline earth metals, ammonia and organic amines as, for example, salts in which the cations are sodium, potassium, magnesium, calcium, or the protonated amines such as those derived from ethylamine, triethylamine, ethanolamine, diethylamino-ethanol, ethylenediamine, piperidine, morpholine, 2-piperidinoethanol, benzylamine, procaine and the like. Thus for example, one can employ miconazole nitrate in place of miconazole free base, or zinc or sodium undecylenate in place of undecylenic acid.

While concentrations will vary with the particular antimycotic agents used, typically concentrations will be from about 0.01% to about 5% range. For example, the preferred concentration of miconazole nitrate is about 2% while that for clotrimazole is from about 1 % and that for acrisorcin is about 0.2%.

The topically effective antimycotic agent can be presented in a variety of forms within the formulation matrix; *i.e.*, it can be dissolved, dispersed, suspended, solubilized, coated, entrapped, or encapsulated. Normally it is dissolved or dispersed.

The formulation matrix comprises at least one polyol/(meth)acrylic copolymer. These are commercially available anionic, cationic, or neutral crosslinked polymers containing as one comonomer an acrylic and/or methacrylic acid including acrylate esters, methacrylate esters, acrylamides and methacrylamides, *i.e.*, same or different structures of the formula: 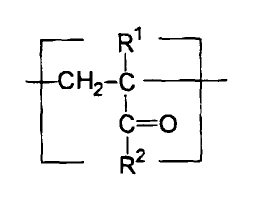 in which R¹ is hydrogen or methyl, preferably hydrogen, and R² is amino, hydroxy, methoxy or ethoxy, which may be further unsubstituted or substituted. Combinations can be employed; *e.g.*, copolymers of acrylic acid with minor levels of long chain alkyl acrylate comonomers crosslinked with allylpentaerythritol.

A second comonomer will be a polyol including alkanediols, alkenediols, and carbohydrates such as 2,4-dihydroxyhexa-1,5-diene, allylpentaerithritol, allylsucrose and the like. Such polyol/(meth)acrylic copolymers are available for example from B. F. Goodrich. Typical of these are Carbopol® 910, Carbopol® 934, Carbopol® 940, Carbopol® 941, Carbopol® 971, Carbopol® 974, Carbopol® 980, Carbopol® 1342, Carbopol® 1382, Carbopol® 2984, Carbopol® ETD 2001, Carbopol® ETD 2020, Carbopol® ETD 2050, Pemulen TR 1, and Pemulen TR 2 (see generally U.S. Drug Master Files 153, 6,542, 7,170, 7,618, 7,757, and 10,072 and USP 23/NF 18 Monograph). Particularly preferred is a copolymer of acrylate and C10-30 alkyl acrylate available under the name Carbopol® ETD 2020. Typically the concentration of the polyol/acrylic copolymer will be from about 0.1% to about 10%.

The formulation matrix also comprises a particulate vegetable starch. Suitable vegetable starches include tapioca starch, corn starch, potato starch, oat starch, rice starch, and wheat starch. Typically the concentration of the vegetable starch will be from about 1% to about 35%.

The formulation matrix also comprises a topically acceptable alcohol. Suitable alcohols include branched or straight lower alkanols chain containing from 1 to 6 carbon atoms. Representative of such alkanols are ethanol, propanol, isopropanol, butanol. isobutanol, sec-butanol, tert-butanol, pentanol, isopentanol, neopentanol, tert-pentanol, hexanol, and isohexanol. Typically the concentration of the alcohol will be from about 5% to about 50% and the amount of water from about 20% to about 80%.

Optionally an anti-inflammatory steroid can be present. Thus as disclosed in U.S. Patent No. 5,002,938, fungal infections often can be effectively treated with a combination product containing steroids and an antifungal agent, particularly an imidazole. The sensitivity of fungal organisms varies with their life cycles, spores being more resistant to treatment than mycelia. It has been suggested that steroids may induce fungal spores to produce mycelia, thereby making them more sensitive to treatment. Also, steroids are known to produce vasoconstriction at the site of application, thereby delaying or preventing elimination of the antifungal agent from the application site and permitting the antifungal agent to remain in the epidermis for longer periods of time. A locally applied anti-inflammatory agent also offers direct and immediate relief for the inflammatory component of the lesion. Suitable steroids include, but are not limited to triamcinolone, betamethasone, dexamethasone, flunisolide, prednisone, prednisolone, methylprednisolone, fluocinolone, diflorasone, halcinonide, hydrocortisone, desoximetasone (desoxymethasone), diflucortolone, flucloronide (fluclorolone acetonide), fluocinonide, fluocortolone, fluprednidene, flurandrenolide (flurandrenolone), clobetasol, clobetasone, alclometasone, flumethasone, fluocortolone, amcinonide, beclometasone, fluticasone, difluprednate, and desonide. Derivatives of such steroids such as the acetate, tebutate, propionate, butyrate, pivalate, hexanoate, sodium succinate, benzoate, sodium phosphate, valerate, diacetate, dipropionate, hexacetonide, and acetonide, also can de employed. While concentrations will vary with the particular steroid used, typically concentrations will be from about 0.01% to about 1% range. For example, the preferred concentration of triamcinolone is from about 0.025% to about 1% while that for betamethasone is from about 0.01 % to about 0.2%.

In addition, the compositions optionally can contain an effective amount of an antimicrobial agent such as triclosan, benzalkonium chloride, *p*-chlorometaxylenol, chlorhexidine, and the like so as to provide both antifungal and antimicrobial properties. The amount will depend upon the particular agent but generally will be from about 0.5 to about 3%.

The present preparation also can contain other auxiliary components typically employed in topical pharmaceutical compositions. Although amounts will depend on the function of the auxiliary component and are not critical, such auxiliary components when present generally will not exceed a total of about 20% of the entire composition and more commonly will total about 5%. These auxiliary components include, for example, physiologically acceptable acids and bases used in small amounts to adjust pH (as might be indicated to accommodate solubilities of a particular antifungal agent or indeed any active ingredient); buffers to maintain pH such as citric acid-citrate salts, acetic acid-acetate salts, and benzoic acid-benzoic salt systems to maintain pH; surfactants such as cetalkonium chloride and sodium ethasulfate; humectants such as polyethylene glycol and butylene glycol; preservatives; stabilizers such as sodium nitrate; antioxidants such as butylated hydroxytoluene and tocopherol acetate; colorants; denaturants such as brucine and *t*-butyl alcohol; emollients such as cyclomethicone, sorbitan monooleate, and octyl isononanoate; thickeners; liposomes; chelating agents such as ethylenediamine tetraacetic acid; and the like. In some instances, one component may perform several functions, *e.g.*, sorbitan monooleate is both an emollient and an emulsifier; Pemulen® TR-2 resin is both a polymeric emulsifier and a thickener or viscosifier; and cetalkonium chloride is both a surface active agent and an antiseptic.

Compositions containing (*i*) at least one topically effective antimycotic agent in an amount at least sufficient to produce a topical antimycotic effect, *(ii)* a topically acceptable polyol/acrylic copolymer, and *(iii)* a topically acceptable particulate vegetable starch, in *(iv)* an aqueous alcoholic base are applied in a conventional manner to the afflicted skin area to treat mycoses, leaving it with a soothing "powdery" after-feel while producing the desired topical antimycotic effect.

The following example will serve to further typify the nature of the invention but should not be construed as a limitation on the scope of the invention.

| *Example 1* | |
|---|---|
| Ingredients | % W / W |
| *Antifungal agent* | |
| Miconazole Nitrate | 2.10 |

| *Formulation Matrix* | |
|---|---|
| Acrylates/C10-30 Alkyl Acrylate Copolymer Carbopol® ETD 2020) | 0.900 |
| Carbomer 1342 (Pemulen® TR-2) | 0.350 |
| Starch/Acrylates/Acrylamide Copolymer (Waterlock® A-240) | 0.0100 |
| Tapioca Starch | 22.0 |
| Ethanol | 35.56 |
| Purified Water | 28.48 |

| *Auxiliaries* | |
|---|---|
| Brucine Sulfate | 0.00539 |
| Butylated Hydroxytoluene | 0.0500 |
| Butylene Glycol | 5.00 |
| D&C Red No. 33 | 0.00010 |
| Edetate Disodium | 0.0500 |
| Octyl Isononanoate | 5.00 |
| Sodium Hydroxide q.s. pH | 3.9 |
| Sodium Nitrate | 0.100 |
| Sorbitan Monoleate | 0.350 |
| t-Butyl Alcohol | 0.0462 |
| Total | 100.0 |

The ingredients are thoroughly mixed to form a topical lotion.

| *Example 2* | |
|---|---|
| Ingredients | % W/W |
| *Antifungal agent* | |
| Tolnaftate | 1.05 |

| *Formulation Matrix* | |
|---|---|
| Acrylates/C10-30 Alkyl Acrylate Crosspolymer (Pemulen® TR-2) | 0.400 |
| Starch/Acrylates/Acrylamide Copolymer (Waterlock® A-240) | 0.0100 |
| Tapioca Starch | 20.0 |
| Ethyl Alcohol | 28.4 |
| Purified Water | 36.0 |

| *Auxiliaries* | |
|---|---|
| Brucine Sulfate | 0.0045 |
| Butylated Hydroxytoluene | 0.0500 |
| Butylene Glycol | 2.00 |
| D&C Red No. 33 | 0.00010 |
| Edetate Disodium | 0.0500 |
| Cyclomethicone | 12.0 |
| Sodium Hydroxide q.s. | pH 6.0-8.0 |
| Tertiary Butyl Alcohol | 0.0355 |
| Total | 100.0 |

The ingredients are thoroughly mixed to form a topical lotion.

| *Example 3* | |
|---|---|
| Ingredients | % W / W |
| *Antifungal agent* | |
| Miconazole Nitrate | 2.10 |

| *Formulation Matrix* | |
|---|---|
| Acrylates/C10-30 Alkyl Acrylate Crosspolymer (Carbopol® 1382) | 0.800 |
| Acrylates/C10-30 Alkyl Acrylate Crosspolymer (Pemulen® TR-2) | 0.200 |
| Starch/Acrylates/Acrylamide Copolymer (Waterlock® A-240) | 0.0100 |
| Tapioca Starch | 15.0 |
| Ethyl Alcohol | 30.8 |
| Purified Water | 40.293 |

| *Auxiliaries* | |
|---|---|
| Brucine Sulfate | 0.00490 |
| Butylated Hydroxytoluene | 0.0500 |
| Polyethylene Glycol 300 | 5.00 |
| D&C Red No. 33 | 0.00010 |
| Edetate Disodium | 0.0500 |
| Octyl Isononanoate | 5.00 |
| Tocopherol Acetate | 0.500 |
| Sodium Hydroxide q.s. | pH 3.9 |
| Sodium Nitrate | 0.150 |
| Tertiary Butyl Alcohol | 0.0420 |
| Total | 100.0 |

The ingredients are thoroughly mixed to form a topical lotion.

| *Example 4* | |
|---|---|
| Ingredients | % W / W |
| *Antifungal, anti-inflammatory, and antimicrobial agents* | |
| Clotrimazole | 1.05 |
| Triamcinolone | 0.2 |
| Chlorhexidine hydrochloride | 2.0 |

| *Formulation Matrix* | |
|---|---|
| Acrylates/C10-30 Alkyl Acrylate Crosspolymer (Pemulen® TR-2) | 0.400 |
| Starch/Acrylates/Acrylamide Copolymer (Waterlock® A-240) | 0.0100 |
| Corn Starch | 20.0 |
| Ethyl Alcohol | 28.4 |
| Purified Water | 33.8 |

| *Auxiliaries* | |
|---|---|
| Butylated Hydroxytoluene | 0.0500 |
| Butylene Glycol | 2.00 |
| D&C Red No. 33 | 0.00010 |
| Edetate Disodium | 0.0500 |
| Cyclomethicone | 12.0 |
| Sodium Hydroxide q.s. | pH 3.9 |
| Tertiary Butyl Alcohol | 0.0355 |
| Total | 100.0 |

The ingredients are thoroughly mixed to form a topical preparation having anti-inflammatory and antimicrobial properties in addition to antifungal activities.

## Claims

1. A topical antimycotic composition comprising (i) at least one topically effective antimycotic agent in an amount at least sufficient to produce a topical antimycotic effect, (ii) a topically acceptable polyol/acrylic copolymer, and (iii) a topically acceptable particulate vegetable starch, in (iv) an aqueous alcoholic base.

2. A composition according to claim 1 wherein the antimycotic reagent comprises at least one member selected from the group consisting of imidazoles, allylamines, pyridine derivatives, undecylenic acid derivatives, tolnaftate, haloprogin, clioquinol, acrisorcin, iodochlorhydroxyquin, and nystatin, or physiologically acceptable non-toxic salt thereof.

3. A composition according to any one of the preceding claims wherein the concentration of the antimycotic agent is from 0.01% to 5% by weight.

4. A composition according to any one of the preceding claims wherein the antimycotic reagent is miconazole nitrate.

5. A composition according to any one of the preceding claims wherein the topically acceptable polyol/acrylic copolymer is an anionic, cationic or neutral crosslinked polymer of (i) at least one acrylic acid, methacrylic acid, acrylate ester, methacrylate ester, acrylamides or methacrylamides, and (ii) an alkanediol, alkenediol or carbohydrate.

6. A composition according to any one of the preceding claims wherein the vegetable starch is tapioca starch, corn starch, potato starch, oat starch, rice starch or wheat starch.

7. A composition according to any one of the preceding claims wherein the vegetable starch is tapioca starch.

8. A composition according to any one of the preceding claims wherein the aqueous alcoholic base comprises at least one of ethanol, propanol, isopropanol, butanol, isobutanol, sec-butanol, tert-butanol, pentanol, neopentanol, tert-pentanol, hexanol and isohexanol.

9. A composition according to any one of the preceding claims further comprising an effective amount of a topical steroidal anti-inflammatory agent.

10. A composition according to any one of the preceding claims further comprising an effective amount of a topical antimicrobial agent.

11. A composition according to any one of the preceding claims further comprising 1 to 20% by weight of a glycol selected from polyethylene glycol and butylene glycol.

12. A composition according to claim 11 comprising 5% by weight of butylene glycol.

13. A composition according to any one of the preceding claims wherein the antimycotic agent is miconazole nitrate at a concentration of about 2%, the polyol/acrylic copolymer is a copolymer of acrylate and C10-C30 alkyl acrylate, the starch is tapioca starch, and the aqueous alcoholic base comprises ethanol.

14. A composition according to any one of the preceding claims for use in therapy.

15. Use of a composition according to any one of claims 1 to 13 for the preparation of a medicament for the treatment of a mycosis.
